# EUROPEAN PATENT APPLICATION

(11) **EP 2 522 783 A1**
(43) Date of publication of application: **14.11.2012**
(21) Application number: 12166841.2
(22) Date of filing: 04.05.2012
(51) Int. Cl.: E03D 9/00, A47K 5/12, A61L 9/12

(54) **Fragrance dispensing system**

(30) Priority: 09.05.2011 US 201161483880 P; 06.03.2012 US 201213412998
(71) Applicant: ILLINOIS TOOL WORKS INC., Glenview Illinois 60026-1215 (US)
(72) Inventor: Ferree, Steve, Bridgeville, DE Delaware 19933 (US); Fleming, Steven, Allen, MD Maryland 21810 (US)
(74) Representative: Finnie, Peter John

(57) **Abstract**

A fragrance dispensing system (10) includes a bottle (12) having a bottle body (18) with an outwardly protruding upper abutment member and an outwardly protruding lower abutment member. The fragrance dispensing system (10) also includes a mounting base (14) shaped and dimensioned for selective attachment of the bottle (12) thereto and a fragrance band (20) selectively secured about the bottle (12) between the upper abutment member and the lower abutment member.

## Description

### BACKGROUND OF THE INVENTION

### 1. Field of the Invention

The present invention relates to a fragrance dispensing system.

### 2. Description of the Related Art

Soap systems specifically designed for the transportation industry have been available for some time. For example, the assignee of the present application previously provided a soap system including a bottle with a projection at the bottom that is adapted to fit into a base unit or mount which can be attached to a bathroom wall or counter. The projection at the bottom of the soap bottle can either be threaded so as to screw into the corresponding mount, or it can be a bore with inwardly projecting lugs for engagement with hook lock cut outs in order to be snapped into the corresponding mount. In addition, the threaded mount can be converted into a snap mount by means of an adapter. The advantage of this product is the ability to switch out soap quickly and easily (unlike the under-the-counter systems). This soap system is disclosed in U.S. Patent No. 5,148,948, entitled "Liquid Soap Dispenser With Mounting Base".

Other air freshener products, both active and passive, have previously been developed for the transportation industry. These air freshener products have also been designed to meet the specific needs of the airlines with regard to odor counteraction, duration, etc., and patents have been applied for in these areas as well. For example, see U.S. Patent Application Publication Nos. 2008/0296401, entitled "Fragrance Dispenser", and 2008/0257978, entitled "Fragrance Dispenser".

The transportation industries, particularly airlines, frequently seek products that will reduce overall cost, improve odor counteraction, or provide a unique, customizable appearance. To address these needs attempts have been made to combine soap bottles and air fresheners into one product, allowing the airlines to switch out their soap and air freshener at the same time, precluding the need for a separate passive air freshener, and/or regular use of an active air freshener except when needed. The present invention addresses these needs by providing a fragrance dispensing system overcoming prior deficiencies.

### SUMMARY OF THE INVENTION

It is, therefore, an object of the present invention to provide a fragrance dispensing system including a bottle having a bottle body with an outwardly protruding upper abutment member and an outwardly protruding lower abutment member. The fragrance dispensing system also includes a mounting base shaped and dimensioned for selective attachment of the bottle thereto and a fragrance band selectively secured about the bottle between the upper abutment member and the lower abutment member.

It is also an object of the present invention to provide a fragrance dispensing system wherein the fragrance band is a compression molded polymer.

It is another object of the present invention to provide a fragrance dispensing system wherein the fragrance band is impregnated with fragrance oil.

It is a further object of the present invention to provide a fragrance dispensing system wherein the fragrance band is composed of polyethylene.

It is also an object of the present invention to provide a fragrance dispensing system wherein the fragrance band is composed of polypropylene.

It is another object of the present invention to provide a fragrance dispensing system wherein the fragrance band defines an annular body.

It is a further object of the present invention to provide a fragrance dispensing system wherein a circumferential recess is positioned between the upper abutment member and the lower abutment member spaced from the bottom of the bottle and a coupling protrusion extends from the bottom of the bottle.

It is also an object of the present invention to provide a fragrance dispensing system wherein a thickness of the fragrance unit is substantially equal to a depth of the circumferential recess.

It is another object of the present invention to provide a fragrance dispensing system wherein the upper abutment member is a circumferential upper lip and the lower abutment member is a circumferential lower lip between which the fragrance band is positioned when secured to the bottle.

It is a further object of the present invention to provide a fragrance dispensing system wherein the bottle and the fragrance band are resilient.

Other objects and advantages of the present invention will become apparent from the following detailed description when viewed in conjunction with the accompanying drawings, which set forth certain embodiments of the invention.

### BRIEF DESCRIPTION OF THE DRAWINGS

Figure 1A is an exploded perspective view of the dispensing bottle and fragrance unit in accordance with the present invention.
Figure 1B is an assembled perspective view of the dispensing bottle and fragrance unit shown in Figure 1A.
Figure 2 is a partial cross sectional view of the present fragrance dispensing system showing the mounting base and the fragrance band mounting area in cross section.
Figure 3 is a detailed view of the dispensing bottle.
Figure 3A is a detailed view of the dispensing bottle showing the upper lip.
Figure 4A is a cross sectional view along the line A-A in Figure 4B.
Figure 4B is front plan view of the mounting base.

### DESCRIPTION OF THE PREFERRED EMBODIMENT

The detailed embodiment of the present invention is disclosed herein. It should be understood, however, that the disclosed embodiment is merely exemplary of the invention, which may be embodied in various forms. Therefore, the details disclosed herein are not to be interpreted as limiting, but merely as a basis for teaching one skilled in the art how to make and/or use the invention.

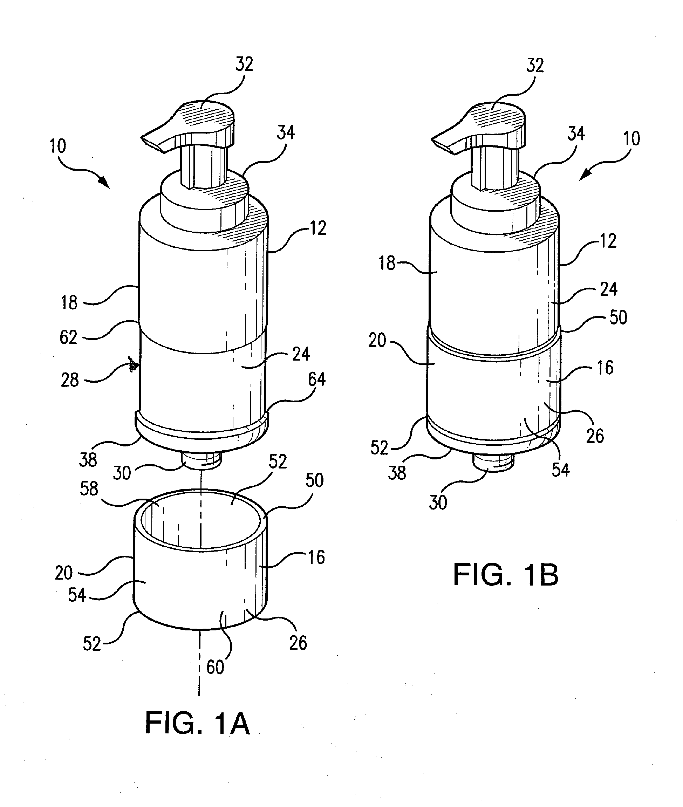

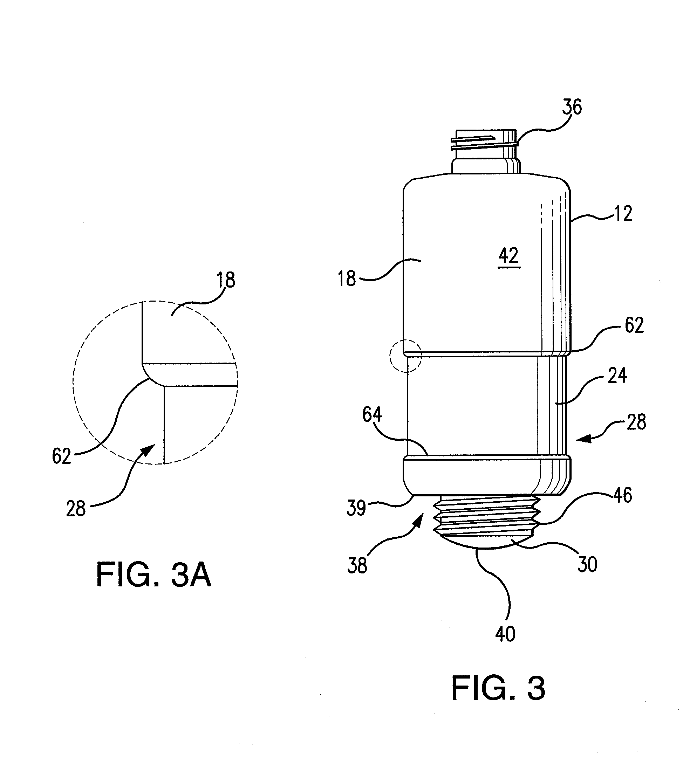

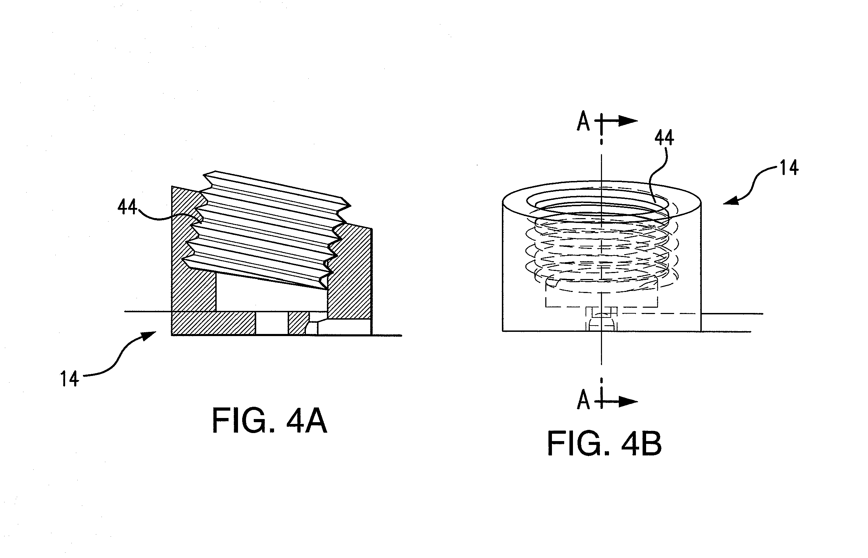

Referring to Figures 1A, 1 B, 2, 3, 3A, 4A and 4B, a fragrance dispensing system 10 is disclosed. The fragrance dispensing system 10 includes a liquid dispensing bottle 12, for example, a soap dispensing bottle, and a mounting base 14. A fragrance unit 16 is secured about the dispensing bottle 12 so as not to interfere with the secure attachment of the dispensing bottle 12 to the mounting base 14. While a soap dispensing bottle is disclosed in accordance with a preferred embodiment, it is appreciated the dispensing bottle may be filled with various liquids requiring controlled dispensing.

The fragrance unit 16, in the form of a fragrance band 20 as discussed below in greater detail, fits onto the dispensing bottle 12. The fragrance unit 16 is composed of a porous and absorbent material saturated with fragrance oil and printed according to customer requests in order to coordinate with soap and/or logo/label. The fragrance saturation is designed to last 4-5 days - the average time that the soap dispensing bottle remains on the aircraft or other location before it is changed out.

The fragrance unit 16 of the present invention is a compression molded polymer, in particular, compression molded polypropylene, polyethylene or equivalent material, that can absorb up to 14 grams of fragrance oil (or other fragrance composition) where previous air fresheners absorbed only about 3.0 grams of fragrance oil. As is appreciated, the higher fragrance loading and the porosity of the compression molded polymer increase the duration of the air freshener properties of the fragrance unit 16 and provide a more uniform fragrance release profile. It is appreciated, the 14 gram loading of the fragrance unit 16 is a maximum determined by the testing of a particular fragrance unit manufactured in accordance with the principles of the present invention. However, it is appreciated the size (thickness, width) of the fragrance unit 16 could be adjusted to absorb more/less fragrance oil. For example, a fragrance unit 16 manufactured in accordance with the present invention was found to permit impregnation of about 6 grams of fragrance oil per fragrance unit 16. Using a thicker fragrance unit 16 impregnation of up to 14 grams of fragrance oil per fragrance unit has been achieved. Ultimately, there is no physical limit with regard to the quantity of fragrance oil that may be impregnated into the fragrance unit, per se, as the limiting factor is the size of the fragrance unit. In accordance with a preferred embodiment, the ratio of the mass of the fragrance oil used in the fragrance unit to the mass of the unsaturated fragrance unit ranges from at least approximately 1.587 : 1 to 1.460 : 1 as shown in Table 1. It is appreciated Table 1 only reflects loading amounts. As such, it is appreciated the compression molded fragrance unit of the present invention has significantly more fragrance release surface area due to the fact that it is composed of micro openings in the plastic matrix.

**TABLE 1**

| **Fragrance Mass to Fragrance Band Mass Ratios** | | | | |
|---|---|---|---|---|
| **Belle-Aire 84543** | | | | |
| | Unsaturated | Saturated | Fragrance Mass | Mass:Mass |
| Trial 1 | 4.250 | 11.190 | 6.940 | 1.633 |
| Trial 2 | 4.405 | 11.198 | 6.793 | 1.542 |
| Trial 3 | 4.224 | 10.922 | 6.698 | 1.586 |
| Average Mass to Mass Ratio | | **1.587** | | |

| **Oriental Aromatics 26690** | | | | |
|---|---|---|---|---|
| | Unsaturated | Saturated | Fragrance | Mass:Mass |
| Trial 1 | 4.213 | 10.668 | 6.455 | 1.532 |
| Trial 2 | 4.314 | 10.842 | 6.528 | 1.513 |
| Trial 3 | 4.423 | 11.004 | 6.581 | 1.488 |
| Average Mass to Mass Ratio | | **1.511** | | |

| **Belle-Aire 85156** | | | | |
|---|---|---|---|---|
| | Unsaturated | Saturated | Fragrance Mass | Mass:Mass |
| Trial 1 | 4.163 | 10.341 | 6.178 | 1.484 |
| Trial 2 | 4.427 | 11.163 | 6.736 | 1.522 |
| Triad 3 | 4.184 | 9.932 | 5.748 | 1.374 |
| Average Mass to Mass Ratio | | **1.460** | | |

The present fragrance dispensing system 10 provides a single component fragrance band 20 that is easy to assemble onto and remove from a dispensing bottle 12. The fragrance dispensing system 10 also provides high fragrance loading on the fragrance unit 16 achieving longer lasting and more effective air freshening properties, provides high fragrance unit porosity achieving a uniform fragrance release profile, and provides a printable and moldable fragrance unit 16 for unique, fine detail, multiple color designs. The fragrance dispensing system 10 further provides an air freshening fragrance unit 16 and dispensing bottle 12 combination to improve customer compliance in changing passive air freshening fragrance units 16 at an appropriate frequency.

The fragrance band 20 is fitted securely to the dispensing bottle 12 for selective attachment and removal. As briefly discussed above, the fragrance band 20 is composed of a compression molded polymer impregnated with fragrance oil. The fragrance band 20 acts as a passive air freshener by delivering the fragrance through evaporation once activated by the customer. The fragrance band 20 can be printed or molded to customer specifications. As will be appreciated based upon the following disclosure, the fragrance band 20 is physically separable from the dispensing bottle 12, so that different fragrance band 20 and dispensing bottle 12 combinations are possible and the fragrance band 20 can easily be removed from the dispensing bottle 12 to permit recycling of the dispensing bottle 12 when it is time to throw out the fragrance band 20. The separate construction also simplifies recycling of the dispensing bottle 12 by allowing the fragrance band 20 to easily be removed from the dispensing bottle 12. The fragrance band 20 fits securely to the dispensing bottle 12 without the use of a housing or adhesive.

The fragrance unit 16 is made by compression (or sinter) molding of a polymer using conventional techniques to create an annular fragrance band 20 that is ultimately secured to the dispensing bottle 12. As is appreciated, compression molding involves the formation of products wherein the molding material, generally preheated, is first placed in an open, heated mold cavity. The mold is closed with a top force or plug member, pressure is applied to force the material into contact with all mold areas, and heat and pressure are maintained until the molding material has cured. In accordance with the present invention, the compression molding process employs thermoplastic resins, for example, polyethylene or polypropylene, in the form of granules in a partially cured stage. Because the granules are not fully melted during the compression molding process, the resulting structure is porous allowing for the impregnation with fragrance oils. As discussed above, the relative porosity and size of the resulting structure is such that each fragrance band 20 is capable of being impregnated with as much as 14 grams of fragrance oil, allowing for extended passive dispensing of the fragrance while the fragrance band 20 is secured about the dispensing bottle 12 and positioned for use. Due to the resulting porous material achieved through compression molding, the fragrance band 20 achieves increased air to fragrance unit contact, resulting in a more uniform fragrance release profile that is not surface area limited.

In accordance with one manufacturing methodology, the fragrance bands are manufactured by first compression molding a planar sheet of compression molded polymer material. The sheet of compression molded polymer material is then cut into strips which are formed into bands and the opposed ends thereof are welded to create the final fragrance bands in accordance with the present invention.

It is appreciated the compression molded polymer from which the fragrance band 20 is composed can be modified to absorb varying amounts of fragrance. Therefore, the fragrance band 20 can be designed to meet specific customer air freshening requirements like fragrance weight loss profile and duration. In addition, the fragrance band 20 can be molded or printed. Printing can include multiple colors, lettering or small details. The fragrance band 20 is separate from the liquid dispensing bottle 12 allowing for various combinations of fragrance band 20 and dispensing bottles 12.

The fragrance band 20 is shaped and dimensioned for a snug fit about the bottle body 18 of the dispensing bottle 12, thereby avoiding the need for the use of an adhesive, housing or secondary device of any kind. This increases the exposed surface area of the fragrance band 20 improving the overall performance of the fragrance band 20 as a passive air freshener. In particular, the fragrance band 20 is annular and includes an inner surface 22 shaped and dimensioned for facing relationship with the outer surface 24 of the bottle body 18 and an outer surface 26 facing away from the dispensing bottle 12. As will be discussed below in greater detail, the fragrance band 20 is shaped and dimensioned to fit within a circumferential recess 28 formed in the body 18 of the dispensing bottle 12. In particular, the fragrance band 20 is shaped and dimensioned for positioning about the bottle body 18 wherein outwardly protruding, upper and lower abutment members 62, 64 formed along the bottler body 18 retain the fragrance band 20 is position thereabout. Because the fragrance band 20 is positioned within a recess 28 and between upper and lower abutment members 62, 64 formed in the bottle body 18 of the dispensing bottle 12, the fragrance band 20 is spaced from the bottom of the dispensing bottle 12 allowing a threaded coupling protrusion 30 of the dispensing bottle 12 to extend through the fragrance band 20 and engage with the mounting base 14.

The fragrance band 20 includes a top edge 50 and a bottom edge 52 with a side wall 54 extending therebetween. The side wall 54 includes the inner surface 22 defined by an inner wall 58 shaped and dimensioned for facing relationship with the outer surface 24 of the dispensing bottle 12 and an outer surface 26 defined by an outer wall 60 facing away from the dispensing bottle 12. The distance between the inner surface 22 and the outer surface 26 is the thickness of the fragrance band 20, which is preferably substantially equal to the depth of the circumferential recess 28 formed in the body of the dispensing bottle 12.

The dispensing bottle 12 is in the form of a liquid dispenser and includes a plastic bottle body 18 that may be refilled. The dispensing bottle 12 includes a conventional manually actuated pump and spout assembly 32 mounted on a cap 34 threadably connected to the neck 36 formed at the top of the bottle body 18. As is appreciated by those skilled in the art, the pump and spout assembly 32 includes a dip tube extending to the bottom of the bottle body 18, whereby measured amounts of liquid, such as soap, hand lotion and the like, can be dispensed from the dispensing bottle 12 by manually pushing downwardly and releasing the pump and spout assembly 32.

The bottom 38 of the dispensing bottle 12 is formed with an integral, cylindrical coupling protrusion 30 having a spherical bottom wall 40. In particular, the coupling protrusion 30 extends downwardly from the bottom 39 of the bottle body 18 and is integrally formed therewith. In accordance with a preferred embodiment, the coupling protrusion 30 is hollow and forms a portion of the inner cavity 42 of the dispensing bottle 12 in which the dispensed liquid is stored. Because of the spherical bottom wall 40, the dispensing bottle 12 cannot support itself in an erect or vertical position on a supporting surface.

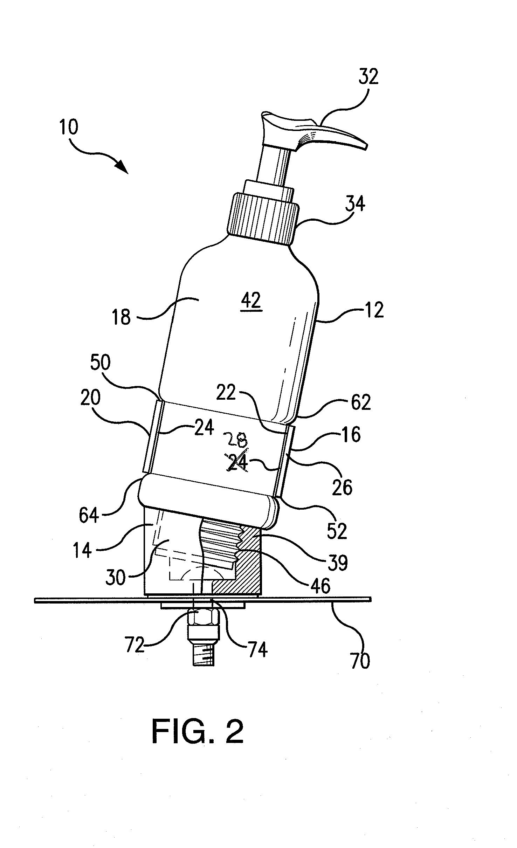

In order that the dispensing bottle 12 can be supported on a horizontal surface, such as a sink counter top 70, shown in Figure 2, a mounting base 14 is provided having internal threads 44 which cooperate with external threads 46 formed on the coupling protrusion 30. In accordance with a preferred embodiment, the mounting base 14 is secured to the countertop 70 by a threaded stud 72 depending from the bottom of the mounting base 14 and extending through a hole 74 provided in the countertop 70. Coupling of the mounting base to the support surface is described in U.S. Patent No. 5,148,948, entitled "Liquid Soap Dispenser With Mounting Base", which is incorporated herein by reference.

From the above description, it will be readily appreciated by those skilled in the art that the liquid soap dispensing bottle 12 and cooperating mounting base 14 of the present invention can be readily mounted on sink countertops as standard equipment, or retrofitted on existing sink countertops. By their construction and arrangement the dispensing bottle 12 cannot be easily removed from the mounting base 14, and if it is removed, the spherical bottom wall 40 formed on the end of the coupling protrusion 30 prevent the dispensing bottle 12 from standing upwardly on a supporting surface, thereby making the dispensing bottle 12 a difficult product to use without the base, and thus discouraging and inhibiting theft of the filled dispensing bottles.

While the present invention is disclosed with reference to a dispensing bottle that screws into a mounting base, it is appreciated the concepts underlying the present invention might be applied in other environments, for example, it might be applied to foaming soap bottles with bayonet type connections and flat bottom bottles that use a spring mechanism to hold the bottle in place.

As briefly discussed above, the bottle body 18 is provided with a circumferential recess 28 shaped and dimensioned for receiving the fragrance band 20. The circumferential recess 28 is defined by the upper abutment member, which is composed of an outwardly protruding, circumferential upper lip 62, and the lower abutment member, which is composed of an outwardly protruding, circumferential lower lip 64 (positioned adjacent the bottom 39 of the bottle body 18), between which the fragrance band 20 is positioned when secured to the dispensing bottle 12. As such, the fragrance band 20 is shaped to have a length dimension, that is, the distance from the top edge 50 to the bottom edge 52, which is slightly smaller than the distance between the upper lip 62 and the lower lip 64. Selective attachment is achieved by shaping the bottle body 18 and the fragrance band 20 such that the outer diameters of the upper lip 62 and the lower lip 64 (that is, lateral extent to which the upper and lower lips 62, 64 extend beyond the outer surface 24 of the bottle body 18 between the upper and lower lips 62, 64) are slightly larger than then inner diameter of the fragrance band 20, while the outer surface 24 of the bottle body 18 between the upper lip 62 and the lower lip 64 has a diameter that is slightly smaller than the inner diameter of the fragrance band 20. As such, and taking advantage of the resilience of the bottle body 18 and the fragrance band 20, the fragrance band 20 may be slid over either the upper lip 62 or the lower lip 64 until the fragrance band 20 moves into position within the circumferential recess 28, and the fragrance band 20, upper lip 62 and/or lower lip 64 resiliently return to their original shape after being slightly biased during the application process. Once this is completed, the fragrance band 20 is held in position by the upper lip 62 and the lower lip 64 bearing against the top edge 50 and bottom edge 52 of the fragrance band 20.

Referring now to Figure 2, a cross sectional view showing the dispensing bottle 12 and fragrance band 20 coupled to a mounting base 14 according to one embodiment of the present invention is presented. As discussed above, the fragrance band 20 has a substantially annular shape and is positioned about and within the circumferential recess 28 of the bottle body 18 of the dispensing bottle 12 with the top edge 50 adjacent an upper lip 62 defining the circumferential recess 28 and the bottom edge 52 positioned adjacent the bottom 39 of the bottle body 18 of the dispensing bottle 12 (but above the threaded coupling protrusion 30 so that dispensing bottle 12 may be screwed into the mounting base 14 in accordance with the present invention).

In the disclosed embodiment, the fragrance band 20 has a substantially annular shape; however, other embodiments may comprise different shapes. For example, it is appreciated the fragrance band 20 might be shaped to conform with the shape of the bottle and could, therefore, have other than a circular cross section shape when viewed from above, that is, when viewed along a plane bisecting the fragrance band 20 perpendicular to a longitudinal axis extending through the center of the annular body. Similarly, the sidewalls of the fragrance band 20 may be formed with various shapes offering desirable aesthetic characteristics.

Although a single fragrance band is employed in accordance with an embodiment disclosed above, it is appreciated the fragrance unit may be composed of two or more fragrance bands shaped and dimensioned for positioning within the circumferential recess.

The present fragrance dispensing system provides advantages over existing systems. The present system provides a one-piece fragrance band that is integrated with a dispensing bottle without the need for adhesive or other cumbersome coupling structures. In addition, the fragrance band, because of its construction and positioning about the dispensing bottle provides highly efficient dispersal of fragrance into the environment. The new fragrance band can be molded providing a three dimensional look.

While the preferred embodiment has been shown and described, it will be understood that there is no intent to limit the invention by such disclosure, but rather, is intended to cover all modifications and alternate constructions falling within the spirit and scope of the invention.

## Claims

1. A fragrance dispensing system, comprising:
a bottle including a bottle body having an outwardly protruding upper abutment member and an outwardly protruding lower abutment member;
a mounting base shaped and dimensioned for selective attachment of the bottle thereto; and
a fragrance band selectively secured about the bottle between the upper abutment member and the lower abutment member.

2. The fragrance dispensing system according to claim 1, wherein the fragrance band is a compression molded polymer.

3. The fragrance dispensing system according to claim 2, wherein the fragrance band is impregnated with fragrance oil.

4. The fragrance dispensing system according to claim 2, wherein the fragrance band is composed of polyethylene.

5. The fragrance dispensing system according to claim 2, wherein the fragrance band is composed of polypropylene.

6. The fragrance dispensing system according to claim 1, wherein the fragrance band defines an annular body.

7. The fragrance dispensing system according to claim 1, wherein a circumferential recess is positioned between the upper abutment member and the lower abutment member spaced from the bottom of the bottle and a coupling protrusion extends from the bottom of the bottle.

8. The fragrance dispensing system according to claim 7, wherein a thickness of the fragrance band is substantially equal to a depth of the circumferential recess.

9. The fragrance dispensing system according to claim 1, wherein the upper abutment member is a circumferential upper lip and the lower abutment member is a circumferential lower lip between which the fragrance band is positioned when secured to the bottle.

10. The fragrance dispensing system according to claim 1, wherein the bottle and the fragrance band are resilient.
